# EUROPEAN PATENT APPLICATION

(11) **EP 2 514 362 A1**
(43) Date of publication of application: **24.10.2012**
(21) Application number: 12150198.5
(22) Date of filing: 04.01.2012
(51) Int. Cl.: A61B 5/1486, A61B 5/145, C12Q 1/00, G01N 33/66, G01N 27/327

(54) **Sensing fluid concentration for continuous glucose monitoring**

(30) Priority: 05.01.2011 US 985224
(71) Applicant: Arkal Medical, Inc., Fremont, CA 94538 (US)
(72) Inventor: Tamada, Janet, Fremont,, CA 94538 (US); Tierney, Michael J, Fremont,, CA 94538 (US); Madabhushi, Ramki, Fremont,, CA 94538 (US); Jina, Arvind N, Fremont,, CA 94538 (US)
(74) Representative: Dee, Ian Mark

(57) **Abstract**

An analyte monitor having a plurality of fluid paths, each fluid path having a distal opening adapted to be disposed on one side of a stratum corneum layer of a user's skin, a proximal opening adapted to be disposed on another side of the stratum corneum layer and an interior space extending between the distal and proximal openings; a sensing zone in fluid communication with the proximal openings of the fluid paths; sensing fluid extending from the sensing zone into substantially the entire interior space of the fluid paths; and an analyte sensor adapted to detect a concentration of analyte in the sensing fluid within the sensing zone, wherein at least one of the sensing fluid and the analyte sensor comprises a catalyst for mutarotation of glucose. The invention also includes a method of using the monitor.

## Description

### INCORPORATION BY REFERENCE

All publications and patent applications mentioned in this specification are herein incorporated by reference to the same extent as if each individual publication or patent application was specifically and individually indicated to be incorporated by reference.

### BACKGROUND OF THE INVENTION

The invention relates to methods and apparatus for monitoring the presence and/or concentration of an analyte or analytes, such as for monitoring the glucose level of a person having diabetes. More specifically, the invention relates to systems, devices, sensors and tools and methods associated therewith for monitoring analyte levels continuously, or substantially continuously.

Diabetes is a chronic, life-threatening disease for which there is no known cure at present. It is a syndrome characterized by hyperglycemia and relative insulin deficiency. Diabetes affects more than 120 million people worldwide, and is projected to affect more than 220 million people by the year 2020. There are 20.8 million children and adults in the United States, or 7% of the population, who have diabetes. Of these people, 14.6 million have been diagnosed with the disease, while unfortunately nearly one-third remain undiagnosed. It is estimated that one out of every three children today will develop diabetes sometime during their lifetime. Diabetes is usually irreversible, and can lead to a variety of severe health complications, including coronary artery disease, peripheral vascular disease, blindness and stroke. The Center for Disease Control (CDC) has reported that there is a strong association between being overweight, obesity, diabetes, high blood pressure, high cholesterol, asthma and arthritis. Individuals with a body mass index of 40 or higher are more than 7 times more likely to be diagnosed with diabetes.

There are two main types of diabetes, Type I diabetes (insulin-dependent diabetes mellitus) and Type II diabetes (non-insulin-dependent diabetes mellitus). Varying degrees of insulin secretory failure may be present in both forms of diabetes. In some instances, diabetes is also characterized by insulin resistance. Insulin is the key hormone used in the storage and release of energy from food.

As food is digested, carbohydrates are converted to glucose and glucose is absorbed into the blood stream primarily in the intestines. Excess glucose in the blood, e.g. following a meal, stimulates insulin secretion, which promotes entry of glucose into the cells, which controls the rate of metabolism of most carbohydrates.

Insulin secretion functions to control the level of blood glucose both during fasting and after a meal, to keep the glucose levels at an optimum level. In a non-diabetic person blood glucose levels are typically between 80 and 90 mg/dL of blood during fasting and between 120 to 140 mg/dL during the first hour or so following a meal. For a person with diabetes, the insulin response does not function properly (either due to inadequate levels of insulin production or insulin resistance), resulting in blood glucose levels below 80 mg/dL during fasting and well above 140 mg/dL after a meal.

Currently, persons suffering from diabetes have limited options for treatment, including taking insulin orally or by injection. In some instances, controlling weight and diet can impact the amount of insulin required, particularly for non-insulin dependent diabetics. Monitoring blood glucose levels is an important process that is used to help diabetics maintain blood glucose levels as near as normal as possible throughout the day.

The blood glucose self-monitoring market is the largest self-test market for medical diagnostic products in the world, with a size of approximately over 53 billion in the United States and $7.0 billion worldwide. It is estimated that the worldwide blood glucose self-monitoring market will amount to $9.0 billion by 2008. Failure to manage the disease properly has dire consequences for diabetics. The direct and indirect costs of diabetes exceed $130 billion annually in the United States - about 20% of all healthcare costs.

There are two main types of blood glucose monitoring systems used by patients: noncontinuous systems, also known as single point, discrete or episodic, and continuous systems. Episodic systems consist of meters and tests strips and require blood samples to be drawn from fingertips or alternate sites, such as forearms and legs (e.g. OneTouch® Ultra by LifeScan, Inc., Milpitas, CA, a Johnson & Johnson company). These systems rely on lancing and manipulation of the fingers or alternate blood draw sites, which can be extremely painful and inconvenient, particularly for children.

Continuous monitoring sensors are generally implanted subcutaneously and measure glucose levels in the interstitial fluid at various periods throughout the day, providing data that shows trends in glucose measurements over a short period of time. These sensors are painful during insertion and usually require the assistance of a health care professional. Further, these sensors are intended for use during only a short duration (e.g., monitoring for a matter of days to determine a blood sugar pattern). Subcutaneously implanted sensors also frequently lead to infection and immune response complications. Another major drawback of currently available continuous monitoring devices is that they require frequent, often daily, calibration using blood glucose results that must be obtained from painful finger-sticks using traditional meters and test strips. This calibration, and re-calibration, is required to maintain sensor accuracy and sensitivity, but it can be cumbersome and inconvenient.

Data from various studies such as the Diabetes Control and Complications trial (DCCT) show that frequent testing of blood glucose levels is essential to improve the quality of life for diabetics. However, most diabetics avoid frequent testing because of the inconvenience, fear, and pain of pricking their fingers or alternate sites to obtain blood samples. Thus there is a need to develop simple glucose monitoring systems that eliminate or minimize these barriers to frequent testing. With some embodiments of the proposed present invention a user or diabetic patient can obtain 20 or more glucose test results over a two or three day period thus allowing frequent measurements on a daily basis.

### SUMMARY OF THE DISCLOSURE

One aspect of the invention provides an analyte monitor having a plurality of fluid paths, each fluid path having a distal opening adapted to be disposed on one side of a stratum corneum layer of a user's skin, a proximal opening adapted to be disposed on another side of the stratum corneum layer and an interior space extending between the distal and proximal openings; a sensing zone in fluid communication with the proximal openings of the fluid paths; sensing fluid extending from the sensing zone into substantially the entire interior space of the fluid paths; and an analyte sensor adapted to detect a concentration of analyte in the sensing fluid within the sensing zone, wherein at least one of the sensing fluid and the analyte sensor comprises a catalyst for mutarotation of glucose. In some embodiments, the fluid paths could each include a tissue piercing element.

In some embodiments, the analyte sensor includes a membrane comprising the catalyst.

In some embodiments, the catalyst for mutarotation of glucose includes a phosphate buffer ion. In such embodiments, the sensing fluid could have a concentration of phosphate buffer ions ranging from 100 mM to 500 mM. In some embodiments, the sensing fluid may include a surfactant, an anti-microbial agent, and/or an anti-clotting agent.

In some embodiments, the catalyst for mutarotation of glucose includes a conjugate base of an organic acid, such as propionic acid, acetic acid, benzoic acid, glycolic acid, histidine acid, imidazole acid, glutamic acid, aspartic acid, guanidine acid, guanidine derivative acid, and alpha-hydroxypyridine acid.

In some embodiments, the catalyst for mutarotation of glucose includes an enzyme mutarotase. In other embodiments, the catalyst for mutarotation of glucose includes a polymeric catalyst, such as a polymer having a phosphate group, a polymer having a phosphate group based salt, a polymer having oxo acid, a polymer having an oxo acid based salt, a polyvinyl sulfonic acid, polyvinyl sulfonic acid based salt, poly styrene sulfonic acid, poly styrene sulfonic based salt, polyvinyl phosphoric acid, polyvinyl phosphoric acid based salt, poly acryloxyethyl phosphoric acid, poly acryloxyethyl phosphoric acid based salt, poly 4-vinyl pyridine and poly diallyldimethylammoniumchloride.

Another aspect of the invention provides a method of in vivo monitoring of an individual's interstitial fluid analyte concentration including the following steps: creating a plurality of fluid paths through a stratum corneum layer of an area of the individual's skin, the fluid paths each comprising a distal end in analyte communication with interstitial fluid of the individual, a proximal end in fluid communication with a sensing zone located outside of the patient's body, an interior space extending between the distal and proximal ends of the fluid path, and a sensing fluid filling substantially the entire interior space; and sensing concentration of glucose in the sensing fluid, the sensing step comprising catalyzing for mutarotation of glucose, such as by using a catalyst in the sensing fluid and/or a catalyst that is part of an analyte sensor in contact with the sensing fluid. The method may also include the step of creating at least one of the fluid paths with a tissue piercing element.

In some embodiments, the method also includes the step of buffering the sensing fluid with a catalyst used in the catalyzing step. In some embodiments, the catalyzing includes converting α-form glucose to β-form glucose.

In various embodiments, catalyzing for mutarotation of glucose includes using a phosphate ion as a catalyst, using a conjugate base of an organic acid (such as, e.g., propionic acid, acetic acid, benzoic acid, glycolic acid, histidine acid, imidazole acid, glutamic acid, aspartic acid, guanidine acid, guanidine derivative acid, and alpha-hydroxypyridine acid) as a catalyst, or using a polymeric catalyst (such as, e.g., a polymer having a phosphate group, a polymer having a phosphate group based salt, a polymer having oxo acid, and a polymer having an oxo acid based salt, a polyvinyl sulfonic acid, polyvinyl sulfonic acid based salt, poly styrene sulfonic acid, poly styrene sulfonic based salt, polyvinyl phosphoric acid, polyvinyl phosphoric acid based salt, poly acryloxyethyl phosphoric acid, poly acryloxyethyl phosphoric acid based salt, poly 4-vinyl pyridine and poly diallyldimethylammoniumchloride.)

### BRIEF DESCRIPTION OF THE DRAWINGS

The novel features of the invention are set forth with particularity in the claims that follow. A better understanding of the features and advantages of the present invention will be obtained by reference to the following detailed description that sets forth illustrative embodiments, in which the principles of the invention are utilized, and the accompanying drawings of which:

FIG. 1 is a perspective view of one embodiment of the analyte monitor of this invention.

FIG. 2 is a cross-sectional view of the analyte monitor shown in FIG. 1 showing tissue piercing elements piercing through the patient's skin.

FIGS. 3 and 4 illustrate embodiments in which the analyte monitor comprises a plurality of calibration fluid reservoirs and a sensing fluid reservoir.

FIG. 5 shows an exploded view of an analyte monitor according to one embodiment of the invention.

FIGS. 6A and 6B are a schematic representative drawing of a three electrode system for use with the analyte sensor of one embodiment of this invention. FIG. 6A shows electrodes on a substrate, and FIG. 6B shows the electrodes and a portion of the substrate covered with a reagent.

FIGS. 7A and 7B are a schematic representative drawing of a two electrode system for use with the analyte sensor of one embodiment of this invention. FIG. 7A shows electrodes on a substrate, and FIG. 7B shows the electrodes and a portion of the substrate covered with a reagent.

FIG. 8 illustrates a graph of different phosphate solutions utilized in detection of glucose concentration.

### DETAILED DESCRIPTION

While many of the exemplary embodiments disclosed herein are described in relation to monitoring glucose levels in people with diabetes, it should be understood that aspects of the invention are useful in monitoring glucose levels in people without diabetes, or for monitoring an analyte or analytes other than glucose. For example, the present invention may be used in monitoring the concentration, or presence, of other analytes such as lactate, acetyl choline, amylase, bilirubin, cholesterol, chorionic gonadotropin, creatine kinase (e.g., CK-MB), creatine, DNA, fructosamine, glutamine, growth hormones, hematocrit, hemoglobin (e.g. HbAlc), hormones, ketones, lactate, oxygen, peroxide, prostate-specific antigen, prothrombin, RNA, thyroid stimulating hormone, troponin, drugs such as antibiotics (e.g., gentamicin, vancomycin), digitoxin, digoxin, drugs of abuse, theophylline, and warfarin. Accordingly, while the invention will be described in connection with glucose monitoring, it should be understood that the invention may be used to monitor other analytes as well.

The present invention provides a significant advance in biosensor and analyte monitoring technology. According to various aspects of the invention, a glucose monitoring system may be constructed to be portable, painless, minimally invasive, self-calibrating, integrated and/or have non-implanted sensors which continuously indicate the user's glucose concentration, enabling swift corrective action to be taken by the patient. The invention may also be used in critical care situations, such an in an intensive care unit to assist health care personnel. The sensor and monitor of this invention may be used to measure any other analyte as well, for example, electrolytes such as sodium or potassium ions. As will be appreciated by persons of skill in the art, the glucose sensor can be any suitable sensor including, for example, an electrochemical sensor or an optical sensor.

One aspect of the invention is a glucose monitor. The glucose monitor may comprise a plurality of tissue piercing elements or fluid paths, a sensing zone in fluid communication with the plurality of tissue piercing elements or fluid paths, one or more calibration reservoirs each adapted to house a calibration fluid and in fluid communication with the sensing zone, and a sensor configured to detect glucose and provide an output indicative of the glucose concentration of the fluid in the sensing zone.

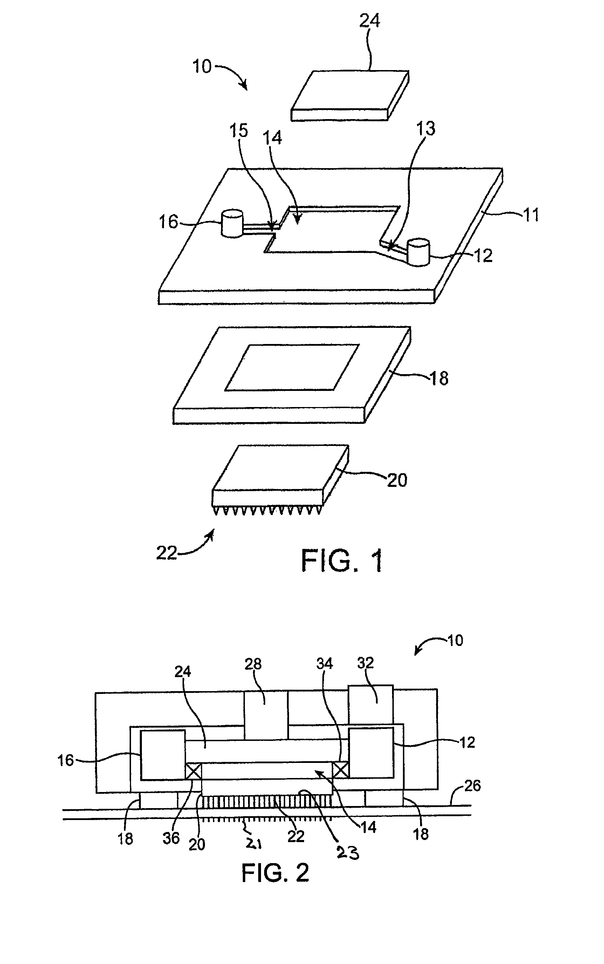

FIGS. 1-2 illustrate one embodiment of the present invention. Glucose monitor 10 includes a fluidic network in which a calibration reservoir 12 is in fluid communication with sensing zone 14 and waste reservoir 16 to allow for the movement of calibration fluids from the reservoirs through sensing zone 14 and into the waste reservoir 16. Glucose monitor 10 includes an adhesive pad or seal 18 which is coupled to substrate or chip 20 which comprises a plurality of tissue piercing elements 22 forming and defining fluid paths.

Glucose monitor 10 includes a sensing layer 11 with a fluidic network having a calibration reservoir 12 in fluid communication with a calibration fluid channel 13 adapted to receive calibration fluid from the calibration fluid reservoir. Calibration fluid channel 13 is in fluid communication with a sensing zone or sensing channel 14. Sensing zone 14 is fluidly connected (optionally via a check valve, not shown) to a waste channel 15 in fluid communication with a waste reservoir 16. As shown, substrate 20 is coupled to an optional adhesive pad 18 for attachment to a user's skin. When in use, the tissue piercing elements 22 each have an interior space defining a fluid path that passes through the stratum corneum 26 of the skin with a distal opening at its distal end 21 in fluid communication with the user's interstitial fluid and a proximal opening at its proximal end 23 in fluid communication with sensing zone 14 and with sensor 24.

While not shown in FIGS. 1-2, at least one pump and at least one check valve can be incorporated into the glucose monitor to facilitate or control the flow of fluid unidirectionally from the calibration fluid reservoir into the sensing zone. Also not shown in FIGS. 1-2 is an actuator which can be manually or automatically actuated and can be configured to work in conjunction with a pump and/or series of valves to initiate the flow of fluid from the calibration fluid reservoir. The channels shown in FIG. 1 are intended to be optional in the glucose monitor, as the calibration fluid can flow directly from the calibration fluid reservoir into the sensing zone (passing through valves), and further directly into the waste reservoir. One or more waste reservoirs may be incorporated into the glucose monitor.

Alternatively, the embodiment in FIG.1 may include a plurality of calibration reservoirs. The calibration reservoirs may include a plurality of calibration fluids. The calibration fluid which may be the sensing fluid, if, for example, the calibration fluid does not include glucose.

In one embodiment, sensing zone 14 and the tissue piercing elements or fluid paths 22 are pre-filled with sensing fluid prior to the first use of the device. The sensing fluid may also filled upon application to the user's skin. Thus, when the device is applied to the user's skin and the tissue piercing elements or fluid paths may pierce the stratum corneum and the epidermis, there is substantially no net fluid transfer from the interstitial fluid into the tissue piercing elements or fluid paths. Rather, glucose diffuses from the interstitial fluid into the fluid within the tissue piercing elements or fluid paths, as described below.

Exemplary tissue piercing elements or fluid paths that can be used with the present invention include microneedles described in Stoeber et al. U.S. Pat. No. 6,406,638; US Patent Appl. Publ. No. 2005/0171480; and US Patent Appl. Publ. No. 2006/0025717. Tissue piercing elements or fluid paths and microneedles described in co-assigned U.S. Patent Appl. No. 11/642,196, filed Dec. 20, 2006 may also be used. Any other tissue piercing elements or fluid paths or needle arrays that can penetrate into the epidermis layer and allow glucose to diffuse from the interstitial fluid into the sensing zone of the present invention may also be incorporated into the embodiments described herein.

Disposed above and in fluid communication with sensing zone 14 is sensor 24. In some embodiments, the sensor is an electrochemical glucose sensor that generates an electrical signal (current, voltage or charge) whose value depends on the concentration of glucose in the fluid within sensing zone 14. Details of sensor 24 are discussed in more detail below.

Electronics element 28 is configured to receive an electrical signal from sensor 24. In some embodiments, electronics element 28 uses the electrical signal to compute a glucose concentration and display it. In other embodiments, electronics element 28 transmits the electrical signal, or information derived from the electrical signal, to a remote device, such as through wireless communication. Electronics element 28 can comprise other electrical components such as an amplifier and an A/D converter which can amplify the electrical signal from the sensor and convert the amplified electrical signal to a digital signal before, for example, determining a glucose concentration or transmitting the signal to an external device which can then determine a glucose concentration.

Glucose monitor 10 can be held in place on the patient's skin by one or more adhesive pads 18.

The glucose monitor has a built-in calibration system. As shown in FIG. 1, the glucose monitor includes one or more calibration reservoirs each adapted to house a calibration fluid. The one or more calibration reservoirs are in fluid communication with the sensing zone. A glucose monitor with two or more calibration fluids can have a sensor that can be calibrated at two or more different glucose concentrations, which allows for a multi-point calibration curve during the sensor calibration. This can provide a more accurate calibration curve which in turn can enable a more accurate glucose concentration determination.

The calibration fluids in each of the different calibration fluid reservoirs have known glucose concentrations, and can be different known glucose concentrations. For example, in some embodiments a first calibration fluid in a first calibration fluid reservoir has a glucose concentration of between about 0 mg/dl and about 100 mg/dl, and a second calibration fluid in a second calibration fluid reservoir has a glucose concentration of between about 100 mg/dl and about 400 mg/dl. The ranges of glucose concentrations in the different calibration fluid reservoirs may, however, be different. When more than one calibration fluid reservoir is used, the calibration fluids in each reservoir may have, however, substantially the same or similar glucose concentrations.

In some embodiments, one of the reservoirs can be filled with a sensing or washing fluid which does not comprise glucose and which is not used to calibrate the glucose sensor. The sensing or washing fluid can comprise, for example, de-ionized water, buffer, surfactants and preservative. More information about the sensing fluid is provided later in the description. In embodiments in which there are two reservoirs and one comprises sensing fluid and the other comprises calibration fluid, the calibration fluid may have a glucose concentration between about 0 mg/dl and about 400 mg/dl, and is used to generate a one-point calibration curve for the sensor. In some embodiments, however, the glucose monitor comprises two or more calibration fluids reservoirs in addition to a sensing or washing fluid reservoir.

Monitoring a subject's interstitial fluid glucose concentration is further described. The method can include calibrating the glucose sensor with one or more different calibrating fluids with different known glucose concentrations. A calibration fluid of known glucose concentration is moved into the sensing zone. This can be done, for example, during manufacture of the monitor, prior to the first use by the patient, or any subsequent time when it may be desirable to recalibrate the sensor. The glucose sensor senses glucose in the calibration fluid in the sensing zone and generates an output signal associated with the known glucose concentration. This information can be used to calibrate (or recalibrate) operation of the glucose sensor.

In some embodiments, any actuating technique described herein may then be used to move an optional second calibrating fluid with a second known glucose concentration from a second calibration fluid reservoir into the sensing zone, displacing the first calibration fluid into the waste area. The sensor then senses the glucose from the second calibration fluid in the sensing zone and generates an output signal associated with the second known glucose concentration. Using these one or more at least two associations of known glucose concentration to glucose sensor output, a calibration curve or plot can be used to associate glucose concentration to the output of the glucose sensor, which can then be used to determine glucose concentration of the glucose that diffuses into the sensing zone from the patient's interstitial fluid. Any number of calibration fluids, and thus calibration points, can be used to calibrate the glucose sensor. The calibrated sensor is then ready to sense glucose in the sensing zone which has diffused from the patient's interstitial fluid.

Describing the method in relation to FIG. 2, upon manual or automatic actuation of actuator 32, fresh calibration fluid is forced from calibration fluid reservoir 12 (only one reservoir is shown) through check valve 34, such as a flap valve, into sensing zone 14. Any fluid within the sensing zone is generally displaced through second check valve 36 into waste reservoir 16. Check valves or similar gating systems can also be used to prevent contamination.

It may be advantageous to retain a calibration fluid with the lower glucose concentration (such as a first concentration between about 0 mg/dl and 100 mg/dl) in the sensing zone after the calibrating step, to provide for faster response times for the glucose sensing. In the method described above where a second calibration fluid has a higher glucose concentration, it may be advantageous to move a volume of the fresh first lower concentration calibration fluid into the sensing zone after the glucose sensor has been calibrated. This would move the second sensing fluid from the sensing zone into waste reservoir. Alternatively, calibrating can comprise calibrating the sensor with a calibration fluid with a higher glucose concentration followed by calibrating the sensor with a calibration fluid with a lower glucose concentration.

Glucose monitors with more than one or more calibration reservoirs have been described. In such embodiments, at least one reservoir can be adapted to house a sensing or washing fluid which does not have any glucose, such as, for example, a buffer, preservative, or de-ionized water. As used herein, "sensing fluid" and "washing fluid" may be used interchangeably. Sensing fluid as used herein can be a special case of calibrating fluid with zero glucose concentration. Sensing fluid can be used to displace calibration fluid from the sensing zone after the calibration step. Glucose would then diffuse from the patient's interstitial fluid into the sensing fluid which does not contain glucose.

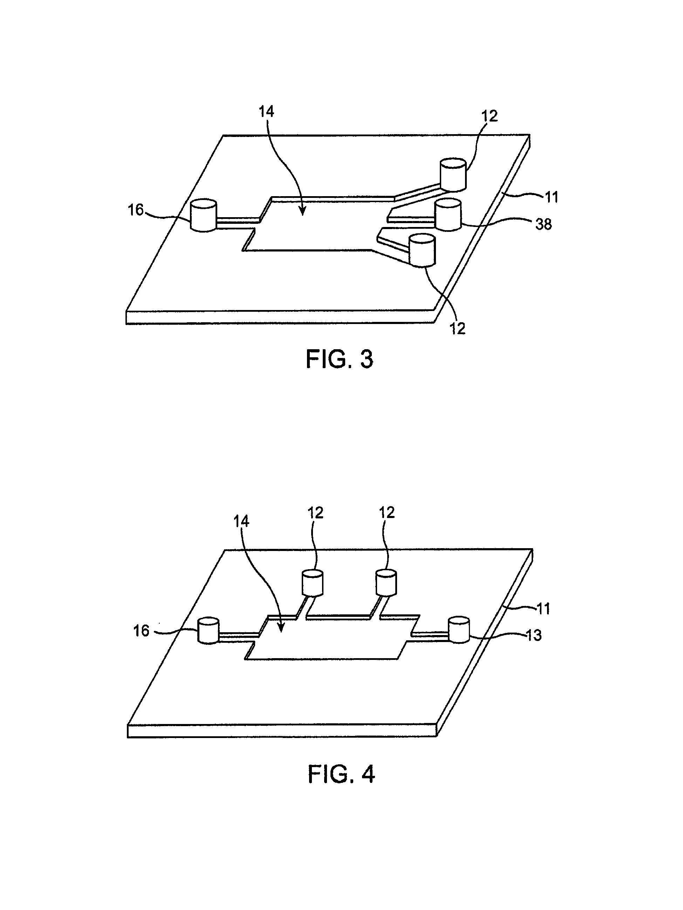

Embodiments in which there are a plurality of calibration fluid reservoirs as well as at least one sensing fluid reservoir are shown in FIGS. 3 and 4. In FIG. 3, glucose monitor 10 is shown comprising two calibration fluid reservoirs 12 and one sensing fluid reservoir 38. All three reservoirs are in fluid communication with the sensing zone. An actuator or actuators (not shown in FIG. 3 and 4) can be configured to move fresh fluid from the reservoirs into the sensing zone.

In some embodiments the sensor is calibrated with any number of calibration fluids as described herein. The actuator can then move sensing fluid from a sensing fluid reservoir into the sensing zone, displacing a calibration fluid. In other embodiments, the sensor may be calibrated with one calibration fluid and then sensing fluid may be moved into the sensing zone, followed by a second calibration fluid being moved into the sensing zone, displacing the sensing fluid and calibrating the sensor with the second calibrating fluid. Fresh sensing fluid can then be actuated into the sensing zone, readying the monitor for diffusion and glucose detection. In this method, there is a "wash" step between calibrating the sensor with fluids of different known glucose concentrations.

In some embodiments at least one finger-stick calibration may optionally be performed or may be required to be performed at any point during the use of the monitors described herein.

Waste reservoirs may be or include an absorption device such as a wicking material to absorb waste fluids. In such embodiments the waste reservoir may not necessarily be an enclosed structure, but may simply be a wicking material or substance in fluid communication with the sensing zone so that it can wick waste fluids as they are moved from the sensing zone.

While in some embodiments the glucose monitor may be manually actuated to initiate the calibrating procedure, the glucose monitor can also be self-calibrating or self-actuating. For example, the glucose monitor can include a programmable component, such as a timer, that is programmed to automatically activate an actuator, such as a pump and valve system, to initiate the flow of fresh fluid from any of the fluid reservoirs into the sensing zone. The timer can be preprogrammed, or in some embodiments the monitor also includes a remote device that is separate from the sensor that can display a glucose concentration. The remote device can be adapted such that it can program the programmable component. For example, a patient may want to program the monitor to calibrate itself at certain times during the day. The monitor can include a timer that can be programmed, reprogrammed by the patient, and/or automatically reprogrammed. The remote device can be adapted for manual programming.

In some embodiments the glucose monitor includes a body and sensing zone temperature sensor, which is more fully described in co-assigned U.S. Patent Appl. No. 11/642,196, filed Dec. 20, 2006.

In some embodiments the glucose monitor includes a vibration assembly adapted to ease the penetration of the needle into the stratum corneum of the skin. Description of exemplary vibration assemblies are described in co-assigned U.S. Patent Appl. No. 11/642,196, filed Dec. 20, 2006.

In some embodiments the monitor can include an applicator to apply the sensor pad or adhesive pad to the skin. The applicator may be part of the sensor device or when the monitor includes separate components, it may be included in any of the different components. The applicator may also be a separate component.

In some embodiments, the tissue piercing elements or fluid paths, fluid reservoirs, sensing zone, sensor, and optional adhesive pads are contained within a sensing structure separate from a reusable structure comprising the electronics element and actuator. This configuration permits the sensing structure, comprising the sensor, sensing fluid and tissue piercing elements or fluid paths to be discarded after a period of use (e.g., when the fluid reservoirs are depleted) while enabling the reusable structure comprising the electronics and actuator to be reused. A flexible covering (made, e.g., of polyester or other plastic-like material) may surround and support the disposable structure. In particular, the interface between an actuator and a fluid reservoir permits the actuator to move fluid out of the reservoir, such as by deforming a wall of the reservoir or forcing the fluid out of the reservoir using a pressurized mechanism, such as a piston. In these embodiments, the disposable sensing structure and the reusable structure may have a mechanical connection, such as a snap or interference fit. Any of the monitor components described herein may, however, be located in the reusable structure or the sensing structure. For example, the tissue piercing elements or fluid paths could be configured to be located in the reusable structure. As another example, one or more fluid reservoirs may be located in the reusable structure and may be refillable, emptiable or separately replaceable from other disposable structures.

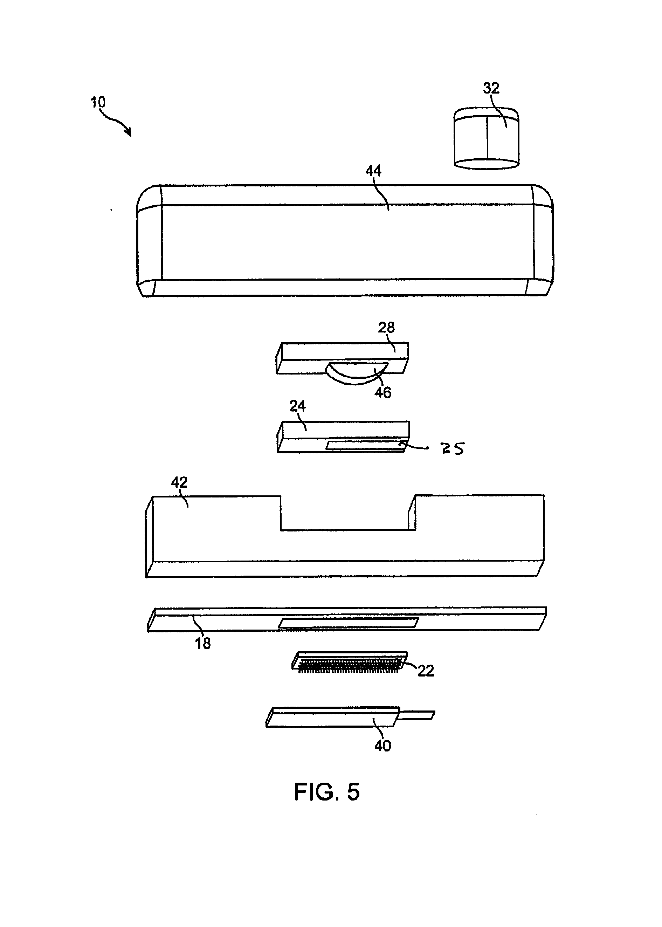

FIG. 5 shows an exploded view of another embodiment of the invention. This figure shows a removable seal 40 covering the distal end of tissue piercing elements or fluid paths 22 and attached, e.g., by adhesive. Removable seal 40 retains the fluid within the tissue piercing elements or fluid paths and sensing zone prior to use and is removed prior to placing the glucose monitor 10 on the skin using adhesive seal 18. In this embodiment, tissue piercing elements or fluid paths 22, the fluid and waste reservoirs, sensing zone 14 and sensor 24 are contained within and/or supported by sensing structure 42 which can be a disposable portion of the monitor. Reusable structure 44 comprises or supports electronics element 28 and actuator 32 that can be used to move sensing fluid out of the fluid reservoirs, through the sensing zone into the waste reservoir. Electrical contacts 46 extend from electronics element 28 to make contact with, for example, electrodes in glucose sensor 24 when the device is assembled.

The following is a description of glucose sensors that may be used with the glucose monitors of this invention. In 1962, Clark and Lyons proposed the first enzyme electrode (that was implemented later by Updike and Hicks) to determine glucose concentration in a sample by combining the specificity of a biological system with the simplicity and sensitivity of an electrochemical transducer. The most common strategies for glucose detection are based on using either glucose oxidase or glucose dehydrogenase enzyme.

Electrochemical sensors for glucose, based on the specific glucose oxidizing enzyme glucose oxidase, have generated considerable interest. Several commercial devices based on this principle have been developed and are widely used currently for monitoring of glucose, e.g., self testing by patients at home, as well as testing in physician offices and hospitals. The earliest amperometric glucose biosensors were based on glucose oxidase (GOX) which generates hydrogen peroxide in the presence of oxygen and glucose according to the following reaction scheme:
Glucose + GOX-FAD (ox) → Gluconolactone + GOX-FADH₂ (red)
GOX-FADH₂ (red) + O₂ → GOX-FAD (ox) + H₂O₂.

Electrochemical biosensors are used for glucose detection because of their high sensitivity, selectivity and low cost. In principal, amperometric detection is based on measuring either the oxidation or reduction of an electroactive compound at a working electrode. A constant potential is applied to that working electrode with respect to another electrode used as the reference electrode. The glucose oxidase enzyme is first reduced in the process but is reoxidized again to its active form by the presence of any oxygen resulting in the formation of hydrogen peroxide. Glucose sensors generally have been designed by monitoring either the hydrogen peroxide formation or the oxygen consumption. The hydrogen peroxide produced is easily detected at a potential of 0.0 volts, 0.1 volts, 0.2 volts, or any other fixed potential relative to a reference electrode such as a Ag/AgCl electrode. However, sensors based on hydrogen peroxide detection are subject to electrochemical interference by the presence of other oxidizable species in clinical samples such as blood or serum. On the other hand, biosensors that monitor oxygen consumption are affected by the variation of oxygen concentration in ambient air or in any of the fluids used with the monitors as described herein. In order to overcome these drawbacks, different strategies have been developed and adopted.

Selectively permeable membranes or polymer films have been used to suppress or minimize interference from endogenous electroactive species in biological samples. Another strategy to solve these problems is to replace oxygen with electrochemical mediators to reoxidize the enzyme. Mediators are electrochemically active compounds that can reoxidize the enzyme (glucose oxidase) and then be reoxidized at the working electrode as shown below:
GOX-FADH₂ (red) + Mediator (ox) → GOX-FAD (ox) + Mediator (red).

Organic conducting salts, ferrocene and ferrocene derivatives, ferricyanide, quinones, and viologens are considered good examples of such mediators. Such electrochemical mediators act as redox couples to shuttle electrons between the enzyme and electrode surface. Because mediators can be detected at lower oxidation potentials than that used for the detection of hydrogen peroxide the interference from electroactive species (e.g., ascorbic and uric acids present) in clinical samples such as blood or serum is greatly reduced. For example ferrocene derivatives have oxidation potentials in the +0.1 to 0.4 V range. Conductive organic salts such as tetrathiafulvalene-tetracyanoquinodimethane (TTF-TCNQ) can operate as low as 0.0 Volts relative to a Ag/AgCl reference electrode. Nankai et al., WO 86/07632, published Dec. 31, 1986, discloses an amperometric biosensor system in which a fluid containing glucose is contacted with glucose oxidase and potassium ferricyanide. The glucose is oxidized and the ferricyanide is reduced to ferrocyanide. This reaction is catalyzed by glucose oxidase. After two minutes, an electrical potential is applied, and a current caused by the re-oxidation of the ferrocyanide to ferricyanide is obtained. The current value, obtained a few seconds after the potential is applied, correlates to the concentration of glucose in the fluid.

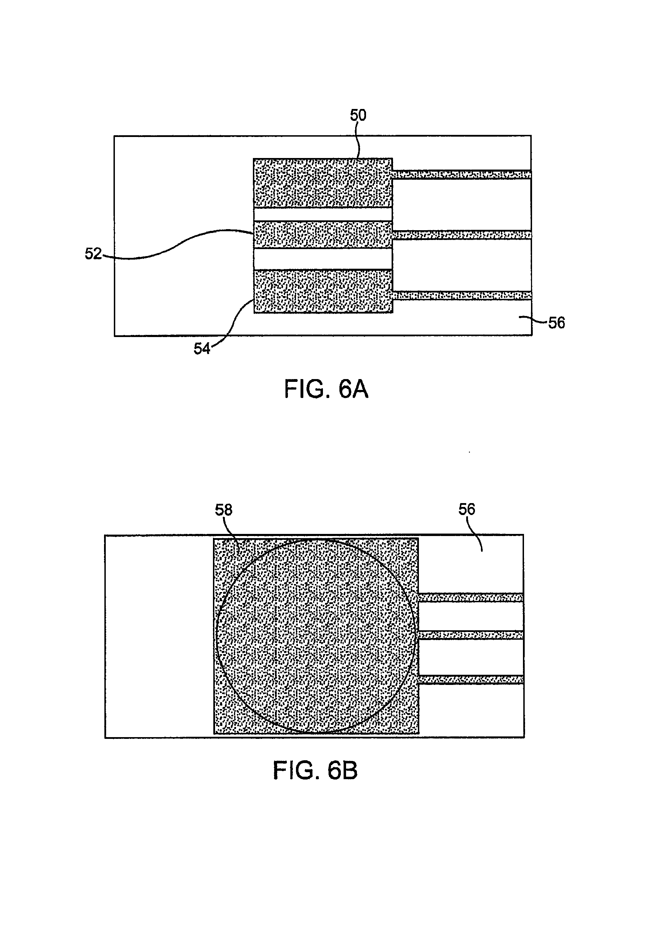

There are multiple glucose sensors that may be used with this invention. In a three electrode system, shown in FIGS. 6A and 6B a working electrode 50, such as Pt, C, or Pt/C is referenced against a reference electrode 52 (such as Ag/AgCl) and a counter electrode 54, such as Pt, provides a means for current flow. The three electrodes are mounted on an electrode substrate 56 as shown in FIG. 6A, then covered with a reagent 58 as shown in FIG. 6B.

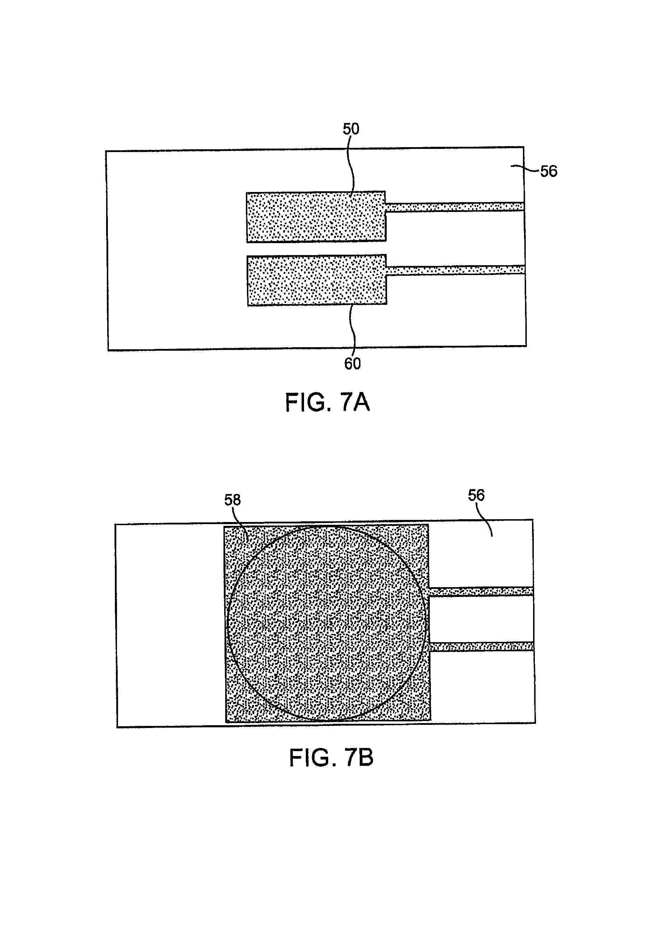

FIGS. 7A and 7B show a two electrode system, wherein the working and auxiliary electrodes, 50 and 60 respectively, are made of different electrically conducting materials. Like the embodiment of FIGS. 6A and 6B, the electrodes are mounted on a flexible substrate 56 (FIG. 7A) and covered with a reagent 58 (FIG. 7B). In an alternative two electrode system, the working and auxiliary electrodes are made of the same electrically conducting materials, where the reagent exposed surface area of the auxiliary electrode is slightly larger than that of the working electrode or where both the working and auxiliary electrodes are substantially of equal dimensions.

In amperometric and coulometric biosensors, immobilization of the enzymes is also very important. Conventional methods of enzyme immobilization include covalent binding, physical adsorption or cross-linking to a suitable matrix may be used. In some embodiments the reagent chemistry can be deposited away from the electrodes using various different dispensing methods.

The glucose sensor can be constructed by immobilizing glucose oxidase enzyme on top of the electrode by using a proprietary cross linker and a coating membrane. The cross linker will hold the enzyme on top of the sensor, and the thin layer membrane (e.g., Nafion, cellulose acetate, polyvinyl chloride, urethane etc) will help the long term stability of the glucose sensor. In the presence of oxygen the glucose oxidase will produce hydrogen peroxide. The hydrogen peroxide can be readily oxidized at the working electrode surface in either two or three electrodes systems.

In some embodiments, the reagent is contained in a reagent well in the biosensor. The reagent includes a redox mediator, an enzyme, and a buffer, and covers substantially equal surface areas of portions of the working and auxiliary electrodes. When a sample containing the analyte to be measured, in this example glucose, comes into contact with the glucose biosensor the analyte is oxidized, and simultaneously the mediator is reduced. After the reaction is complete, an electrical potential difference is applied between the electrodes. In general the amount of oxidized form of the redox mediator at the auxiliary electrode and the applied potential difference must be sufficient to cause diffusion limited electrooxidation of the reduced form of the redox mediator at the surface of the working electrode. After a short time delay, the current produced by the electrooxidation of the reduced form of the redox mediator is measured and correlated to the amount of the analyte concentration in the sample. In some cases, the analyte sought to be measured may be reduced and the redox mediator may be oxidized.

These elements are satisfied by employing a readily reversible redox mediator and using a reagent with the oxidized form of the redox mediator in an amount sufficient to insure that the diffusion current produced is limited by the oxidation of the reduced form of the redox mediator at the working electrode surface. For current produced during electrooxidation to be limited by the oxidation of the reduced form of the redox mediator at the working electrode surface, the amount of the oxidized form of the redox mediator at the surface of the auxiliary electrode exceeds the amount of the reduced form of the redox mediator at the surface of the working electrode. Importantly, when the reagent includes an excess of the oxidized form of the redox mediator, as described below, the working and auxiliary electrodes may be substantially the same size or unequal size as well as made of the same or different electrically conducting material or different conducting materials. From a cost perspective the ability to utilize electrodes that are fabricated from substantially the same material represents an important advantage for inexpensive biosensors.

As explained above, the redox mediator must be readily reversible, and the oxidized form of the redox mediator must be of sufficient type to receive at least one electron from the reaction involving enzyme, analyte, and oxidized form of the redox mediator. For example, when glucose is the analyte to be measured and glucose oxidase is the enzyme, ferricyanide or quinone may be the oxidized form of the redox mediator. Other examples of enzymes and redox mediators (oxidized form) that may be used in measuring particular analytes by the present invention are ferrocene and or ferrocene derivative, ferricyanide, and viologens. Buffers may be used to provide a preferred pH range from about 4 to 8. In one embodiment, the pH range is from about 6 to 7. The buffer may be phosphate (e.g., potassium phosphate) and may be in a range from about 0.01M to 0.5M, such as about 0.05M. (These concentration ranges refer to the reagent composition before it is dried onto the electrode surfaces.) More details regarding glucose sensor chemistry and operation may be found in: Clark L C and Lyons C, "Electrode Systems for Continuous Monitoring in Cardiovascular Surgery," Ann NY Acad Sci, 102:29, 1962; Updike S J, and Hicks G P, "The Enzyme Electrode," Nature, 214:986, 1967; Cass, A. E. G., G. Davis. G. D. Francis, et. al. 1984. Ferrocene--mediated enzyme electrode for amperometric determination of glucose. Anal. Chem. 56:667-671; and Boutelle. M. G., C. Stanford. M. Fillenz, et al. 1986. An amperometric enzyme electrode for monitoring brain glucose in the freely moving rat. Neurosci lett. 72:283-288.

With the above overview, other aspects of glucose or other analyte monitoring devices will be described. The analyte monitor may comprise a plurality of fluid paths or tissue piercing elements, each fluid path or tissue piercing elements comprising a distal opening, a proximal opening and an interior space extending between the distal and proximal openings. The analyte monitor may also comprise a sensing zone in fluid communication with the proximal openings of the fluid paths or tissue piercing elements.

One aspect of the invention relates to the composition of the sensing fluid that fills the sensing zone and into which glucose or other analyte is collected after it diffuses from the skin through the fluid paths or tissue piercing elements. The sensing fluid may extend from the sensing zone into substantially the entire interior space of the fluid paths or tissue piercing elements. The sensing fluid may be pre-filled or filled after application on the user's skin.

In one embodiment, the sensing fluid may comprise a catalyst for mutarotation of glucose. One such mutarotation catalyst is phosphate ions. The phosphate ions may be in a form of a phosphate buffer solution.

The phosphate buffer concentration may range from 100 mM to 500 mM. A high concentration of phosphate buffer is desirable. A high concentration of phosphate provides a greater degree of buffering capacity. A high concentration reduces or eliminates any changes of pH of sensing fluid due to other compounds being extracted into the sensing fluid, or products of the sensing reaction. Phosphate ions can also act as a catalyst for the mutarotation of glucose, which will enable more rapid conversion of the α-form of glucose to the β-form which is subsequently oxidized by the glucose oxidase enzyme. A phosphate concentration of 300 mM (total concentration of all forms of phosphates) is desirable as well.

The "conversion" may be mutarotation. Mutarotation is the interconversion between two different anomeric forms of a molecule. Sugars, such as glucose, generally have cyclic structures. A ring can open and before closing again, the terminal bond can rotate so that when it re-forms the ring, one stereocenter has changed. Glucose, in aqueous solutions, may be approximately 65% in the beta-form and 35% in the alpha form. Interconversion (mutarotation) between the two forms occurs continuously. The rate that the mutarotation occurs can be increased by the presence of catalysts such as phosphate ions. The glucose oxidase enzyme reacts only with the beta form for rapid sensing of glucose in a sample and rapid mutarotation of the remaining alpha glucose to beta may occur. A rapid mutarotation rate is beneficial to maximize sensor signal, as well as to reduce sensor lag time.

Other examples of mutarotation catalysts are conjugate bases of organic acids, such as propionic, acetic, benzoic, and glycolic acids, histidine, imidazole, glutamic acid, aspartic acid, guanidine (and guanidine derivatives), and alpha-hydroxypyridine. An enzyme mutarotase may also be added to the solution or otherwise into the sensing chemistry to catalyze the mutarotation of glucose. Polymeric catalysts, such as polymers comprising phosphate groups or polymer salts, polymers comprising other oxo acids, and their salts, may also be used. These polymeric catalysts may be dissolved in the sensing fluid, or added otherwise to the sensing chemistry. Some examples of polymeric catalysts are polyvinyl sulfonic acid (or its salts), poly styrene sulfonic acid (or its salts), polyvinyl phosphoric acid (or its salts), poly acryloxyethyl phosphoric acid (or its salts), poly 4-vinyl pyridine, polyvinylimidazole, polyhistidine, and poly diallyldimethylammoniumchloride.

The sensing fluid also may comprise a concentration of chloride. A chloride concentration that is isotonic or nearly isotonic with physiological fluid (i.e., interstitial fluid) in a person's body is also desirable. Having an isotonic chloride concentration results in the following benefits. One of the benefits is that there is good fluid communication between the interstitial fluid and the sensing fluid in the sensing zone through the fluid paths or tissue piercing elements. Another benefit is that isotonic chloride concentration maintains a relatively constant chloride concentration in the sensing fluid in order to have a steady reference electrode potential. When the chloride concentration is identical to that in the interstitial fluid, there is no concentration gradient between the body and the sensing zone. Thus, chloride ions may not diffuse from one compartment to the other. The reference electrode potential (which is dependent upon the chloride ion concentration) will then tend to remain stable. The concentration of chloride may range from 100 mM to 160 mM.

The sensing fluid may also comprise a concentration of both phosphate and chloride. The sensing fluid may further comprise a wide variety of solutions. The solutions may comprise sufficient ions to act as an electrolyte for an amperometric biosensor to function.

Other components may be added to the sensing fluid including, but not limited to: 1) surfactants to ensure good wetting of the fluid path or tissue piercing element surface and lumens by both interstitial fluid and sensing fluid, 2) anti-microbials to decrease or prevent the growth of microbes while in storage or while in use, and 3) anti-clotting agents to decrease or prevent the clotting response from closing the fluid paths or tissue piercing element lumens. Some examples of anti-microbial agents are undecylenic acid (and its salts), parabens and also quaternium salts. Some examples of surfactants are detergents, fatty acid salts, fatty alcohols, polyethyleneglycol (PEG) -containing molecules, polysorbates. Further details of the use of anti-clotting agents may be found, e.g., in commonly owned and concurrently filed US patent application entitled, "Flux Enhancement In Continuous Glucose Monitoring," the disclosure of which is incorporated herein by reference.

In another embodiment, the mutarotation catalyst is part of the glucose sensor chemistry instead, or in addition to, being part of the sensing fluid. For example, the in such embodiments the catalyst may be part of the sensor chemistry membrane 25 shown in FIG. 5. In one such embodiment, a polymeric catalyst may be cross-linked together with the GOx enzyme (and possibly other polymers) to form the sensor membrane 25.

The glucose monitor may also comprise a glucose sensor adapted to detect a concentration of glucose in the sensing fluid within the sensing zone.

Further, another method of *in vivo* monitoring of an individual's interstitial fluid glucose concentration will be described. The method comprises creating a plurality of fluid paths through a stratum corneum layer of an area of the individual's skin or inserting tissue piercing elements through the stratum corneum layer of an area of the individual's skin. The tissue piercing elements may be solid or hollow.

The fluid paths may be created by piercing the user's skin. The fluid paths may also be created by removing layers of the individual's skin or by placing holes or pores through the individual's skin. Further, the fluid paths may be created with laser, abrasion or electroporation.

The fluid paths or tissue piercing elements each comprise a distal end in fluid communication with interstitial fluid of the individual, a proximal end in fluid communication with a sensing zone located outside of the patient's body, an interior space extending between the distal and proximal ends of the fluid path, and a sensing fluid filling substantially the entire interior space. In some embodiments of the method, the sensing fluid comprises a phosphate buffer and a chloride concentration.

The method may comprise having a chloride concentration of the sensing fluid that is isotonic or nearly isotonic with the interstitial fluid. Because it is expected that there is fluid communication between the physiological fluid (interstitial fluid) and the fluid in the sensing zone through the tissue piercing elements or fluid paths, and because it is desirable to maintain a relatively constant chloride concentration in the sensing fluid in order to maintain a steady reference electrode potential, formulating the sensing fluid concentration so that the chloride concentration is isotonic with the interstitial fluid is beneficial. When the chloride concentration is identical (or nearly identical) to that in the interstitial fluid, there is no concentration gradient between a person's skin and the sensing zone. Thus, no or little chloride will diffuse from one compartment to the other.

Yet further, the method may comprise sensing a glucose concentration of the sensing fluid.

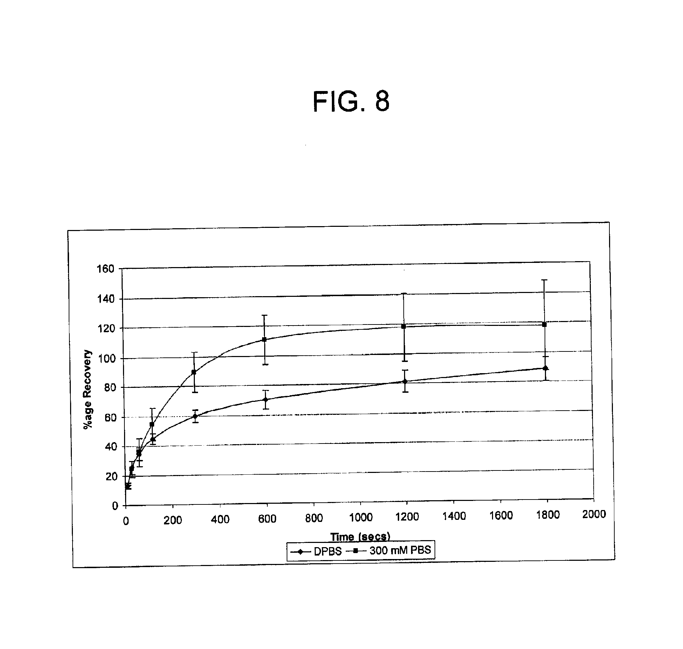

FIG. 8 illustrates a graph of different phosphate solutions utilized in detection of glucose concentration. In FIG. 8, a sample glucose monitoring cell is flushed with a 0.5 mg/dL solution of glucose in either Dulbecco's buffer with 9.5 mM phosphate ions or 300 mM phosphate PBS (phosphate buffer solution). Here, four glucose sensors per condition (i.e., Dulbecco's and 300 mM phosphate) were flushed with the solution of both glucose and phosphate four times each.

The glucose sensor signal (i.e., current) was monitored and integrated over time to calculate a total electrical charge collected (which is proportional to the amount of glucose concentration detected). Because the concentration of glucose and the total volume of the cell are known, the total electrical charge is known.

FIG. 8 shows the percent of total charge recovered at different time points. A steeper curve for the 300 mM PBS shows that the higher phosphate concentration increases the rate of mutarotation of glucose, and allows more complete recovery of the charge (i.e., detection of the glucose concentration) at earlier time points. The less steep curve for the Dulbecco's buffer shows that mutarotation of the glucose occurs very slowly with the low concentration of phosphate.

While exemplary embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that methods and structures within the scope of these claims and their equivalents be covered thereby.

## Claims

1. An analyte monitor comprising:
a plurality of fluid paths, each fluid path comprising a distal opening adapted to be disposed on one side of a stratum corneum layer of a user's skin, a proximal opening adapted to be disposed on another side of the stratum corneum layer and an interior space extending between the distal and proximal openings;
a sensing zone in fluid communication with the proximal openings of the fluid paths;
sensing fluid extending from the sensing zone into substantially the entire interior space of the fluid paths; and
an analyte sensor adapted to detect a concentration of analyte in the sensing fluid within the sensing zone,
wherein at least one of the sensing fluid and the analyte sensor comprises a catalyst for mutarotation of glucose.

2. The analyte monitor of claim 1, wherein the analyte sensor comprises a membrane comprising the catalyst.

3. The analyte monitor of claim 1 or 2, wherein the catalyst for mutarotation of glucose comprises a phosphate buffer ion.

4. The analyte monitor of claim 3, wherein the sensing fluid comprises a concentration of phosphate buffer ions ranging from 100 mM to 500 mM.

5. The analyte monitor of claim 1 or 2, wherein the catalyst for mutarotation of glucose comprises a conjugate base of an organic acid.

6. The analyte monitor of claim 5, wherein the conjugate base of an organic acid is selected from a group consisting of propionic acid, acetic acid, benzoic acid, glycolic acid, histidine acid, imidazole acid, glutamic acid, aspartic acid, guanidine acid, guanidine derivative acid, and alpha-hydroxypyridine acid.

7. The analyte monitor of claim 1 or 2, wherein the catalyst for mutarotation of glucose comprises an enzyme mutarotase.

8. The analyte monitor of claim 1 or 2, wherein the catalyst for mutarotation of glucose comprises a polymeric catalyst.

9. The analyte monitor of claim 8, wherein the polymeric catalyst is selected from a group consisting of a polymer having a phosphate group, a polymer having a phosphate group based salt, a polymer having oxo acid, and a polymer having an oxo acid based salt.

10. The analyte monitor of claim 8, wherein the polymeric catalyst is selected from a group consisting of a polyvinyl sulfonic acid, polyvinyl sulfonic acid based salt, poly styrene sulfonic acid, poly styrene sulfonic based salt, polyvinyl phosphoric acid, polyvinyl phosphoric acid based salt, poly acryloxyethyl phosphoric acid, poly acryloxyethyl phosphoric acid based salt, poly 4-vinyl pyridine and poly diallyldimethylammoniumchloride.

11. The analyte monitor of any one of the preceding claims, wherein the sensing fluid comprises a surfactant.

12. The analyte monitor of any one of the preceding claims, wherein the sensing fluid comprises an anti-microbial agent.

13. The analyte monitor of any one of the preceding claims, wherein the sensing fluid comprises an anti-clotting agent.

14. The analyte monitor of any one of the preceding claims, wherein the fluid paths each comprise a tissue piercing element.

15. A method of in vivo monitoring of an individual's interstitial fluid analyte concentration comprising:
creating a plurality of fluid paths through a stratum corneum layer of an area of the individual's skin, the fluid paths each comprising a distal end in analyte communication with interstitial fluid of the individual, a proximal end in fluid communication with a sensing zone located outside of the patient's body, an interior space extending between the distal and proximal ends of the fluid path, and a sensing fluid filling substantially the entire interior space; and
sensing concentration of glucose in the sensing fluid, the sensing step comprising catalyzing for mutarotation of glucose.

16. The method of claim 15 further comprising buffering the sensing fluid with a catalyst from the catalyzing step.

17. The method of claim 15 wherein catalyzing comprises converting α-form glucose to β-form glucose.

18. The method of claim 15, further comprising creating at least one of the fluid paths with a tissue piercing element.

19. The method of claim 15, wherein catalyzing for mutarotation of glucose comprises using a phosphate ion as a catalyst.

20. The method of claim 15, wherein catalyzing for mutarotation of glucose comprises using a conjugate base of an organic acid as a catalyst.

21. The method of claim 20, wherein the conjugate base of an organic acid is selected from a group consisting of propionic acid, acetic acid, benzoic acid, glycolic acid, histidine acid, imidazole acid, glutamic acid, aspartic acid, guanidine acid, guanidine derivative acid, and alpha-hydroxypyridine acid.

22. The method of claim 15, wherein catalyzing for mutarotation of glucose comprises using an enzyme mutarotase as a catalyst.

23. The method of claim 15, wherein catalyzing for mutarotation of glucose comprises using a polymeric catalyst.

24. The method of claim 23, wherein the polymeric catalyst is selected from a group consisting of a polymer having a phosphate group, a polymer having a phosphate group based salt, a polymer having oxo acid, and a polymer having an oxo acid based salt.

25. The method of claim 23, wherein the polymeric catalyst is selected from a group consisting of a polyvinyl sulfonic acid, polyvinyl sulfonic acid based salt, poly styrene sulfonic acid, poly styrene sulfonic based salt, polyvinyl phosphoric acid, polyvinyl phosphoric acid based salt, poly acryloxyethyl phosphoric acid, poly acryloxyethyl phosphoric acid based salt, poly 4-vinyl pyridine and poly diallyldimethylammoniumchloride.
